# EUROPÄISCHE PATENTANMELDUNG

(11) **EP 1 256 570 A1**
(43) Veröffentlichungstag der Anmeldung: **13.11.2002**
(21) Anmeldenummer: 02008062.8
(22) Anmeldetag: 11.04.2002
(51) Int. Cl.: C07C 251/24, C07B 53/00

(54) **Mehrzähnige unsymmetrische Liganden auf Binaphthylbasis**

(30) Priorität: 09.05.2001 DE 10122539
(71) Anmelder: Degussa AG, 40474 Düsseldorf (DE)
(72) Erfinder: Monsees, Axel, Dr., 60487 Frankfurt (DE); Schneider, Carsten, A., Dr., 60486 Frankfurt (DE); Riermeier, Thomas, Dr., 65439 Flörsheim (DE); Dingerdissen, Uwe, Dr., 64342 Seeheim-Jugenheim (DE)
(74) Vertreter: Ackermann, Joachim, Dr., Patentanwalt Dr. Ackermann

(57) **Zusammenfassung**

Die vorliegende Erfindung beinhaltet neue mehrzähnige unsymmetrische Stickstoff-Sauerstoff-haltige Binaphthylderivate der Formeln (I) und (II) und deren Synthese sowie Komplexe dieser Verbindungen mit Metallen und deren Verwendung als Katalysatoren für enantioselektive Transformationen.

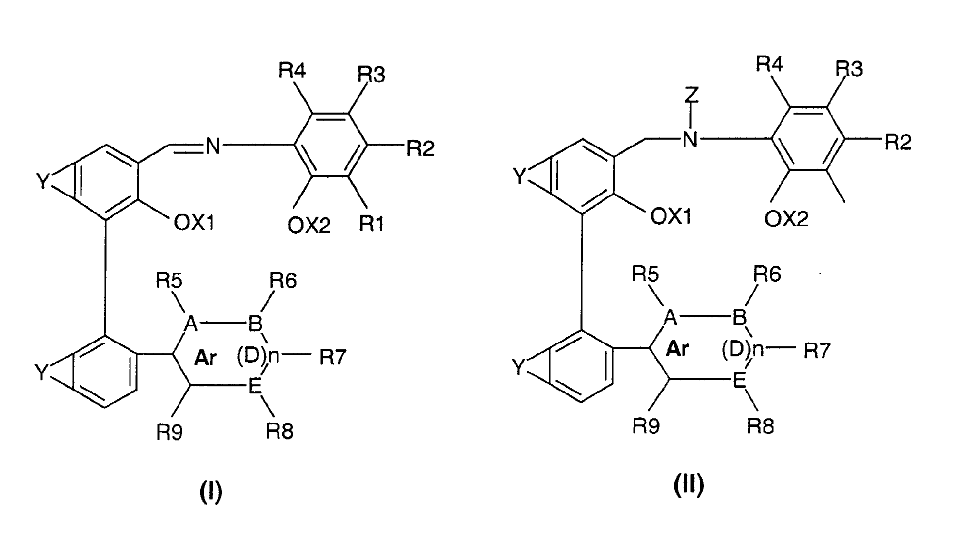

## Beschreibung

Die vorliegende Erfindung beinhaltet neue mehrzähnige unsymmetrische Binaphthylderivate und deren Synthese sowie Komplexe dieser Verbindungen mit Metallen und deren Verwendung als Katalysatoren für enantioselektive Transformationen.

Enantiomerenangereicherte chirale, mehrzähnige Liganden werden in der asymmetrischen Synthese bzw. asymmetrischen Katalyse eingesetzt. Bei der asymmetrischen Katalyse kommt es wesentlich darauf an, daß die elektronischen und die stereochemischen Eigenschaften des Liganden auf das jeweilige Katalyseproblem optimal abgestimmt sind. Durch Variation der Substituenten in solchen Verbindungen lassen sich die elektronischen und sterischen Eigenschaften des Liganden gezielt beeinflussen, so daß Selektivität und Aktivität bei homogenkatalytischen Prozessen gesteuert werden können. Es besteht somit ein großer Bedarf an chiralen Liganden, die sich stereochemisch und elektronisch unterscheiden, um den für eine bestimmte asymmetrische Katalyse optimalen maßgeschneiderten Liganden aufzufinden.

Die Strukturvielfalt der bisher bekannten Sauerstoff/Stickstoff-Liganden (N/O-Liganden) ist sehr groß. Die Gliederung dieser Liganden kann beispielsweise nach Stoffklassen erfolgen. Beispiele für solche Stoffklassen sind Aminoalkohole, Iminoalkohole, Aminoether usw. Diese Einteilung nach Stoffklassen ist insbesondere nützlich, um die elektronischen Eigenschaften der Liganden zu beschreiben.

Darüber hinaus ist eine Klassifizierung von Stickstoff/Sauerstoff-Liganden nach ihren Symmetrieeigenschaften oder nach der Zähnigkeit der Liganden möglich. Diese Strukturierung trägt insbesondere der Stabilität, Aktivität und Stereoselektivität von Metallkomplexen mit Sauerstoff/Stickstoff-Liganden als Katalysatorvorstufen oder als Katalysatoren Rechnung. Neben den weit verbreiteten C₂-symmetrischen Ligandsystemen wie Salen (E. N. Jacobsen et al., *J. Am. Chem. Soc.,* **1990**, *112,* 2801) rücken u. a. unsymmetrische N/O-Liganden immer mehr in den Fokus der asymmetrischen Katalyse. Wichtige Beispiele sind die große Klasse der chiralen N/O-Liganden wie z.B. (1 R,2S)-(+)-*cis*-1-Amino-2-indanol (M. Wills et al., *J. Org. Chem.,* **1997,** *62,* 5226), Schiff-Base-Liganden (A. H. Vetter und A. Berkessel, *Tetrahedron Lett.,* **1998,** *39,* 1741), *cis*-2-Amino-1-acenaphthenol (A. Sudo, M. Matsumoto, Y. Hashimoto, K. Saigo, *Tetrahedron Asymmetry,* **1995,** *6* (8), 1853) und 1-(2-Methoxy-1-naphthyl)isochinolin (G. Chelucci et al., *Tetrahedron Lett.,* **1999,** *40,* 553).

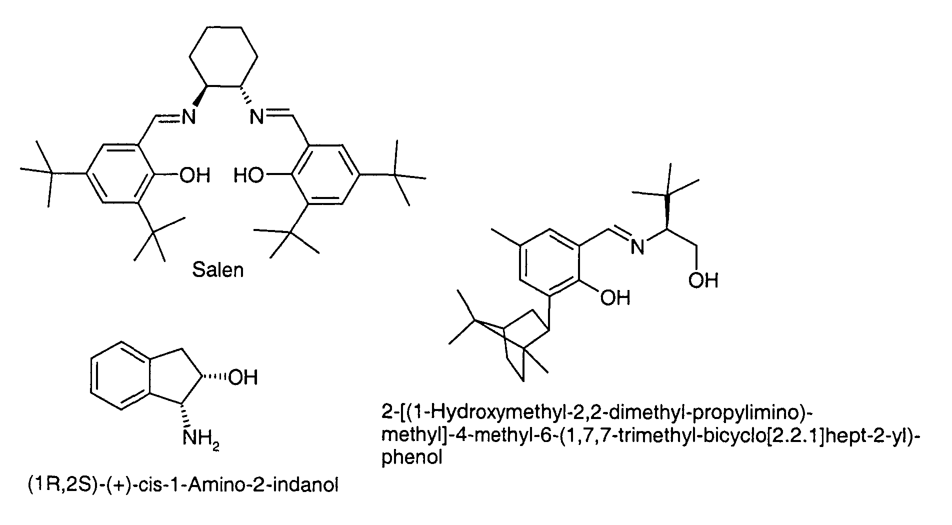

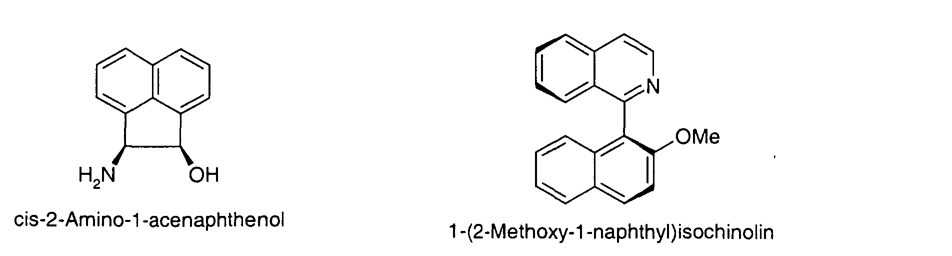

Ein wichtiger Aspekt dieser Verbindungsklassen wird der Schaffung einer besonders asymmetrischen Umgebung des Metallzentrums durch diese Ligandsysteme zugeschrieben. Um eine solche Umgebung für eine effektive Übertragung der Chiralität zu nutzen, ist es vorteilhaft, die Flexibilität des Ligandsystems als inhärente Limitierung der asymmetrischen Induktion zu kontrollieren.

Der vorliegenden Erfindung liegt davon ausgehend die Aufgabe zugrunde neuartige, unsymmetrische, mehrzähnige chirale Stickstoff/Sauerstoff-Liganden (N/O-Liganden), die sich einfach in ihren sterischen und elektronischen Eigenschaften einfach über weite Bereiche variieren lassen, bereitzustellen.

Die Aufgabe wird durch eine Klasse unsymmetrischer mehrzähniger N/O-Liganden der Formeln (I) und (II) gelöst.

Die vorliegende Erfindung betrifft somit Verbindungen der allgemeinen Formeln (I) und (II), 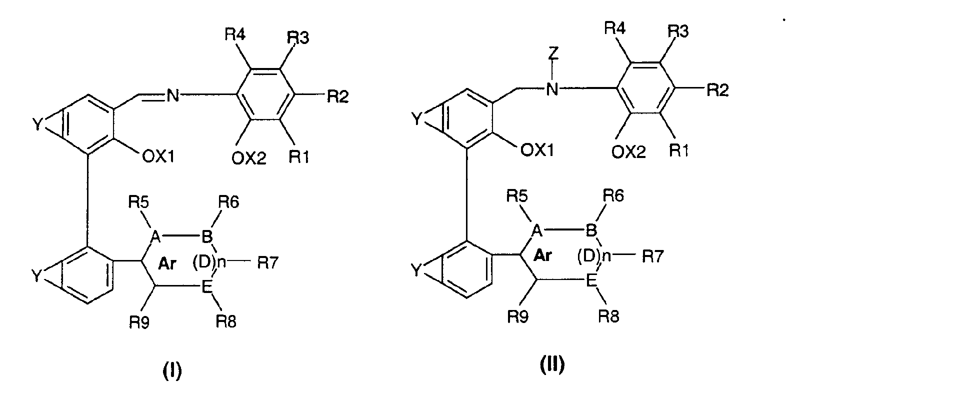 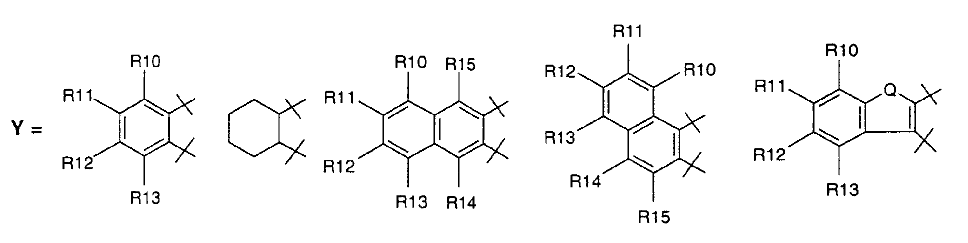 worin n 0 oder 1 sein kann und
- Ar: Teil eines sechsgliedrigen aromatischen oder 5-6 gliedrigen heteroaromatischen Ringsystems ist, wobei das heteroaromatische Ringsystem entweder 1-3 Stickstoffatome oder 1 Sauerstoffatom oder 1 Schwefelatom an den Positionen A, B, D, E enthalten kann. Der sechsgliedrige Heteroaromat ist vorzugsweise ein Pyridyl-Rest, und der fünfgliedrige Heteroaromat ist vorzugsweise ein Furyl-, Thiophenyl- oder Pyrrolyl-Rest.
- R1-R9: unabhängig voneinander ein Wasserstoffatom oder C₁-C₂₀-Alkyl, C₁-C₁₀-Haloalkyl, C₃-C₈-Cycloalkyl, C₅-C₈-Cycloalkenyl, wobei der Cyclus auch 1-2 Heteroatome, ausgewählt aus der Gruppe N, O, S, enthalten kann, C₆-C₁₄-Aryl, Phenyl, Naphthyl, Fluorenyl, C₂-C₁₃-Heteroaryl, wobei die Zahl der Heteroatome, ausgewählt aus der Gruppe N, O, S, 1-4 betragen kann, wobei die cyclischen aliphatischen oder aromatischen Reste bevorzugt 5 bis 7 gliedrige Ringe sind, wobei die vorgenannten Gruppen wiederum ein- oder mehrfach substituiert sein können, wobei diese Substituenten unabhängig voneinander Wasserstoff, C₁-C₂₀-Alkyl, C₂-C₂₀-Alkenyl, C₁-C₁₀ Haloalkyl, C₃-C₈-Cycloalkyl, C₃-C₈-Cycloalkenyl, C₂-C₉-Heteroalkyl, C₁-C₉-Heteroalkenyl, C₆-C₈-Aryl, Phenyl, Naphthyl, Fluorenyl, C₂-C₆ Heteroaryl, wobei die Zahl der Heteroatome, aus der Gruppe N, O, S, 1-4 betragen kann, C₁-C₁₀-Alkoxy, C₁-C₉-Trihalomethylalkyl, Trifluormethyl, Trichlormethyl, Fluoro, Chloro, Bromo, lodo, Cyano, C₁-C₈-Alkyl oder C₆-Aryl darstellt, oder Tri-(C₁-C₆)-Alkylsilyl sein können, und wobei zwei dieser Substituenten verbrückt sein können, bevorzugt so verbrückt, dass eine 5-7 gliedriger aromatischer oder cyclischaliphatischer Rest vorliegt.
- Z: kann Wasserstoff, C₁-C₂₄-Alkyl, Benzyl, C₆-C₈-Aryl, Phenyl, Naphthyl, Allyl, oder Vinyl sein,
- X1, X2: können unabhängig voneinander Wasserstoff, Methyl, Ethyl, Methoxymethyl, Methoxyethyl sein,
- Q: kann Sauerstoff, Schwefel oder Stickstoff sein,
- R10-R15: können unabhängig voneinander ein Wasserstoffatom oder C₁-C₂₀-Alkyl, C₁-C₁₀-Haloalkyl, C₃-C₈-Cycloalkyl, C₅-C₈-Cycloalkenyl, wobei der Cyclus auch 1-2 Heteroatome, ausgewählt aus der Gruppe N, O, S, enthalten kann, C₆-C₁₄-Aryl, Phenyl, Naphthyl, Fluorenyl, C₂-C₁₃-Heteroaryl, wobei die Zahl der Heteroatome, ausgewählt aus der Gruppe N, O, S, 1-4 betragen kann, wobei die cyclischen aliphatischen oder aromatischen Reste bevorzugt 5 bis 7 gliedrige Ringe sind, wobei die vorgenannten Gruppen selbst jeweils ein- oder mehrfach substituiert sein können, diese Substituenten können dabei unabhängig voneinander Wasserstoff, C₁-C₂₀-Alkyl, C₂-C₂₀-Alkinyl, C₂-C₂₀-Alkenyl, C₁-C₁₀-Haloalkyl, C₃-C₈-Cycloalkyl, C₃-C₈-Cycloalkenyl, C₂-C₉-Heteroalkyl, C₁-C₉-Heteroalkenyl, C₆-C₈-Aryl, Phenyl, Naphthyl, Fluorenyl, C₂-C₆-Heteroaryl, wobei die Zahl der Heteroatome aus der Gruppe N, O, S 1-4 betragen kann, C₁-C₁₀-Alkoxy, C₁-C₉-Trihalomethylalkyl, Trifluormethyl, Trichlormethyl, Fluoro, Chloro, Bromo, lodo, Cyano, Carboxylato der Formen COOH und COOM' wobei M' entweder ein einwertiges Kation oder C₁-C₄-Alkyl darstellt, C₁-C₆-Acyloxy, Sulfinato, C₁-C₈-Alkyl oder C₆-Aryl darstellt, oder Tri-(C₁-C₆)-Alkylsilyl sein können.

Die Erfindung betrifft ferner Komplexverbindungen, die ein derartiges, chirales Ligandsystem der Formeln (I) und (II) mit mindestens einem Metall enthalten.

Besonders vorteilhaft ist der modulare Aufbau dieser Ligandensysteme, die eine große Breite an sterischen und elektronischen Variationen zulassen, die durch einfach einführbare Modifikationen erhältlich sind. Durch diese Variationsbreite kann der Ligand jeweils auf ein spezielles Katalyseproblem optimiert werden, um die Selektivität und Aktivität des Katalysators zu steigern. Ein weiterer Vorteil dieser Liganden ist, dass sie eine hoch asymmetrische Koordinationsphäre in einem Metallkomplex schaffen können.

Vorzugsweise weisen die erfindungsgemäßen Liganden unabhängig voneinander als R1-R15 neben Wasserstoff, Alkyl-, Alkenyl-, Cycloalkyl-, Alkoxy- oder Trialkylsilyl-Substituenten auf, die bevorzugt bis zu 10, insbesondere bevorzugt bis zu 6 Kohlenstoffatome besitzen.

Aus der Gruppe der Alkyl- und Alkoxysubstituenten sind Methyl, Ethyl, n-Propyl, 1-Methylethyl, n-Butyl, 1-Methylpropyl, 1,1-Dimethylethyl, n-Pentyl, 1-Methylbutyl, 2-Methylbutyl, 3-Methylbutyl, 2,2-Dimethylpropyl, 1-Ethylpropyl, n-Hexyl, 1,1-Dimethylpropyl, 1,2-Dimethylpropyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1 -methylpropyl, n-Heptyl, n-Octyl, n-Nonyl, n-Decyl, Methoxy, Ethoxy, 1-Propoxy, 2-Propxy, 1-Butoxy, 2-Butoxy, 1,1-Dimethyl-ethoxy besonders bevorzugt.

Unter den cyclischen Alkylsubstituenten sind besonders bevorzugt substituierte und unsubstituierte Cyclopentyl-, Cyclohexyl- und Cycloheptyl-Reste.

Als Alkenylreste sind besonders bevorzugte Reste Vinyl, Propenyl, Isopropenyl, 1-Butenyl, 2-Butenyl, 1-Pentenyl, 2-Pentenyl, 2-Methyl-1-butenyl, 2-Methyl-2-butenyl, 3-Methyl-1-butenyl, 1-Hexenyl, 1-Heptenyl, 2-Heptenyl, 1-Octenyl oder 2-Octenyl.

Unter den cyclischen Alkenylsubstituenten sind besonders bevorzugt Cyclopentenyl, Cyclohexenyl, Cycloheptenyl und Norbornyl.

Unter Arylsubstituenten in R1-R15 sind besonders Phenyl, 2-Methyl-phenyl, 3,5-Dimethyl-phenyl, 4-Methyl-phenyl, 4-Methoxy-phenyl, 3,5-Bis-(trifluormethyl)-phenyl, 4-Trifluormethyl-phenyl, 3,5-Dimethyl-4-Methoxy-phenyl, 4-Dialkyamino, 2-Alkylphenyl, 3-Alkylphenyl, 4-Alkylphenyl, 2,6-Dialkylphenyl, 3,5-Dialkylphenyl, 3,4,5-Trialkylphenyl, 3,5-Bis(trifluormethyl)-phenyl, 4-Trifluormethyl-phenyl, 2-Alkoxyphenyl, 3-Alkoxyphenyl, 4-Alkoxyphenyl, 2,6-Dialkoxylphenyl, 3,5-Dialkoxyphenyl, 3,4,5-Triialkoxyphenyl, 3,5-Dialkyl-4-Alkoxyphenyl bevorzugt, wobei die vorgenannten Alkyl- und Alkoxygruppen jeweils vorzugsweise 1 bis 6 Kohlenstoffatome enthalten.

Alle Haloalkylgruppen weisen vorzugsweise die allgemeinen Formeln CHal₃, CH₂CHal₃, C₂Hal₅ auf, wobei Hal insbesondere für F, Cl und Br stehen kann. Besonders bevorzugt sind Haloalkylgruppen der Formeln CF₃, CH₂CF₃, C₂F₅.

Schließlich sind Ligandsysteme der Formeln (I) und (II) als optisch aktive Ligandsysteme bevorzugt, bei denen ein Enantiomer angereichert ist. Besonders bevorzugt sind Ligandsysteme, bei denen die Enantiomerenanreicherung 90 %, insbesondere 98 % übersteigt.

Die erfindungsgemäße Klasse von mehrzähnigen N/O-Liganden besitzt ein einfach und vielseitig modifizierbares chirales Liganden-Grundgerüst, das sich in Bezug auf seine sterischen und elektronischen Eigenschaften durch einen modularen Aufbau und die leichte Einführbarkeit unterschiedlichster Substituenten sehr breit variieren läßt. N/O-Liganden der Formeln (I) und (II) sind in der Lage in Metallorganischen Komplexen am Metallzentrum eine hochasymmetrische Koordinationssphäre zu schaffen und somit eine effektive asymmetrische Induktion zu ermöglichen. Zusätzlich kann über die einfache Einführbarkeit unterschiedlichster Substituenten in die N/O-Liganden die Flexibilität der Koordinationssphäre des Komplexes sterisch kontolliert werden.
Damit kann für Verbindungen der Formel (I) und (II) ein breites Spektrum von Anwendungen erschlossen werden, da die mehrzähnigen N/O-Liganden sterisch und elektronisch, je nach katalystischem Syntheseverfahren durch die Einführung geeigneter Substituenten optimierbar sind.

Gleichzeitig zeichnen sich die erfindungsgemäßen Verbindungen im Gegensatz zu vielen etablierten Ligandsystemen durch eine besonders einfache synthetische Zugänglichkeit in großer Variationsbreite ausgehend von kommerziell erhältlichen Edukten aus. Dies macht die Liganden der vorliegenden Erfindung leicht zugänglich und somit industriell herstellbar.

Die Auswahl eines entsprechenden Darstellungsverfahrens ist abhängig von der Verfügbarkeit der entsprechenden Edukte und vom gewünschten Substitutionsmuster. Nachfolgend sollen erfindungsgemäße Syntheseverfahren anhand von einfachen Beispielen beschrieben werden, ohne es darauf zu beschränken.

Erfindungsgemäße N/O-Liganden lassen sich wie folgt herstellen:

Ausgehend von einem Binaphthol-Derivat kann in einem geeigneten Lösungsmittel unter Zugabe vom Base und Sulfonierungsreagenz zunächst ein Kupplungsintermediat hergestellt werden, das in einer übergangsmetallkatalysierten Kupplungsreaktion mit einem anderen Kupplungsintermediat verknüpft wird. Eine ortho-Metallierung gefolgt von einer Carbonylierungsreaktion liefert die entsprechende Carbonylzwischenstufe, die im folgenden mit einer Aminkomponente zum erfindungsgemäßen Imin (I) oder durch direkte reduktive Aminierung zum Amin (II) umgesetzt wird. Das Amin (II) ist auch durch Reduktion des entsprechenden Imins (I) zugänglich.

Die eben dargestellten Verfahren werden im folgenden anhand bevorzugter Ausführungsbeispiele näher erläutert.

Das 2-Aryl-2'-hydroxy-1,1'-binaphthyl wird in zwei Stufen analog der Vorschrift (H. Sasaki, R. Irie, T. Hamada, K. Suzuki und T. Katsuki *Tetrahedron* **1994**, *50(41),* 11827-38) hergestellt.

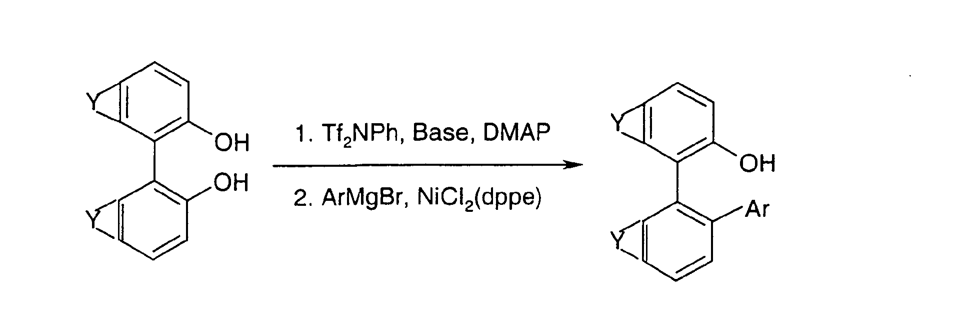

Alternativ ist die Verbindung auch (nach B. Schilling und D. E. Kaufmann, *Eur. J. Org. Chem.* **1998,** *4*, 701-9) über eine Suzuki-Kupplung zugänglich.

Die Carbonylgruppe wird bevorzugt über eine ortho-Metallierung eingeführt. Dazu wird zunächst die Hydroxygruppe in eine ortho-dirigierende Gruppe überführt, mit einer Lithiumbase lithiiert und mit Dimethylformamid carbonyliert. Nach Abspaltung der ortho-dirigierenden Gruppe liegt ein β-Hydroxy-aldehyd vor.

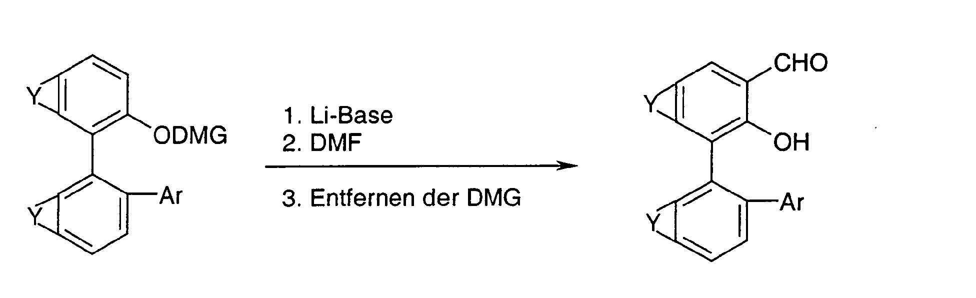

Der Aldehyd kann in großer Variationsbreite mit verschiedenen aromatischen 2-Hydroxy-aminen zu den erfindungsgemäßen Iminen (I) umgesetzt werden. Die Amine (II) können entweder direkt durch reduktive Aminierung aus den β-Hydroxy-aldehyden oder durch Reduktion der Imine (I) erhalten werden.

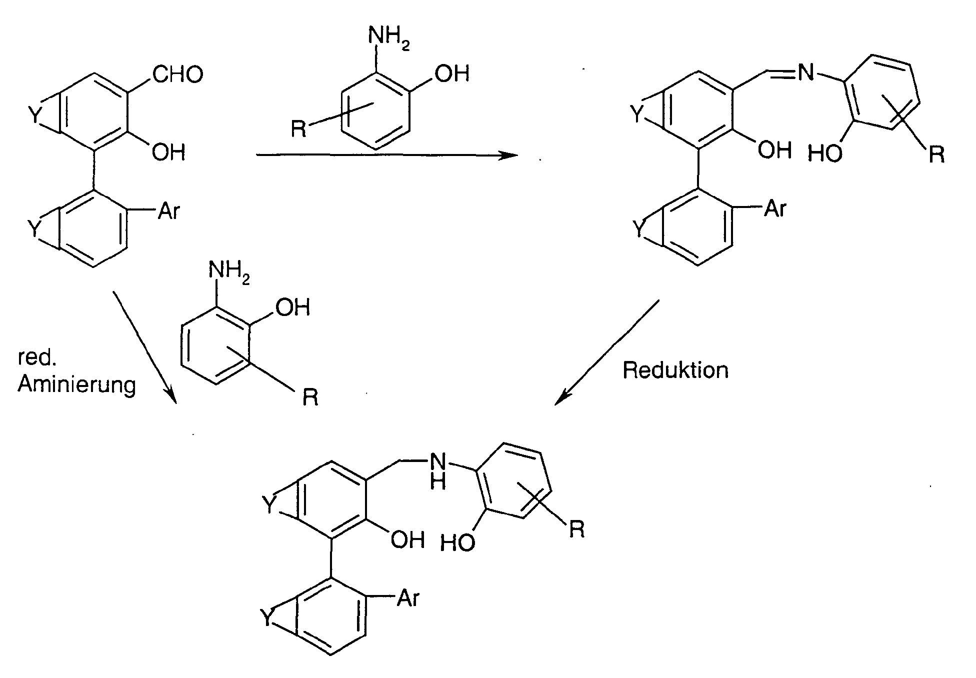

Somit ist ein weiterer Gegenstand der vorliegenden Erfindung ein Verfahren zur Herstellung von Liganden der Formel (I) und (II) umfassend folgende Verfahrensschritte:

### a) Sulfonierung eines 2,2'-Dihydroxy-1,1'-biaromaten der Formel

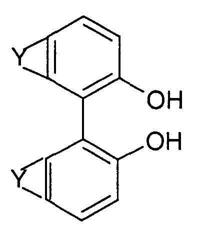

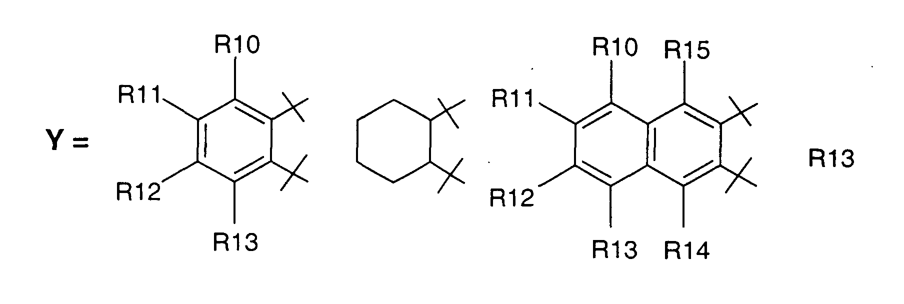

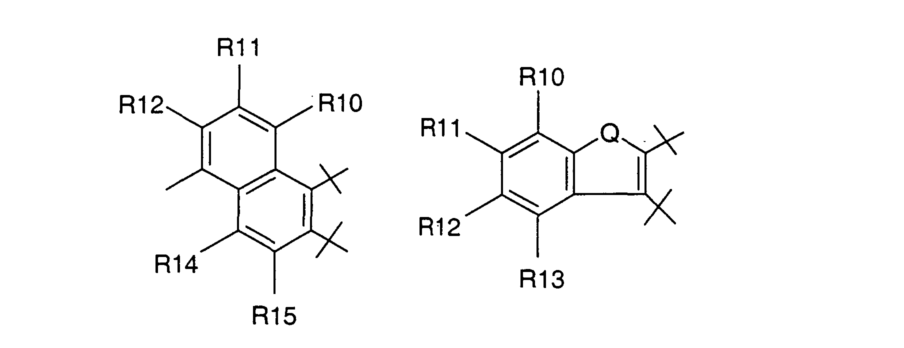

in Anwesenheit einer Base, b) Kupplung des unter a) erhaltenen sulfonierten Produktes mit einer ArMgBr-Verbindung mit 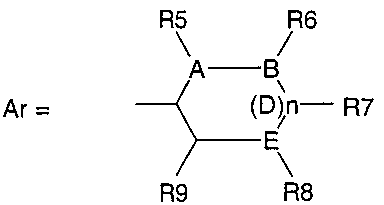 in Gegenwart eines Übergangsmetall-Katalysators, c) Schützung der freien Hydroxygruppen, d) Carbonylierung des unter c) erhaltenen Produktes in ortho-Stellung zur geschützen Hydroxygruppe, e) Entschützung der Hydroxyfunktion und Umsetzung des carbonylierten Produktes aus d) mit einem aromatischen Amin der Formel

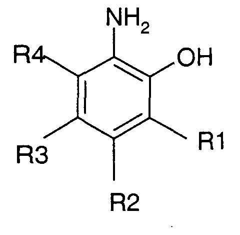

Die Verbindungen der allgemeinen Formeln (I) und (II) können als Liganden an Metallen in Metall-katalysierten Reaktionen (wie z.B. der Reduktion mit anorg. Hydriden, Transferhydrierungen, Hydrosilylierung, Epoxidöffnungen, Epoxidierungen, allylische Oxidationen, Bayer-Villinger Oxidationen, Oxidation von Sulfiden, Cyclopropanierungen, Diels-Alder Reaktionen, Michael-Additionen, Cyanhydrinreaktionen, Strecker und streckerartige Reaktionen, En-Reaktionen, [3+2] Cycloadditionen, Grignard Reaktionen, Addition von Zinkorganylen an Carbonylverbindungen oder Aldol Reaktionen) eingesetzt werden. Sie sind insbesondere für asymmetrische Reaktionen gut geeignet. Insbesondere erweist es sich hier als vorteilhaft, daß sich die Liganden der allgemeinen Formeln (I) und (II) durch ihre einfache, breite Abwandelbarkeit sterisch und elektronisch sehr gut auf das jeweilige Substrat und die katalytische Reaktion abstimmen lassen.

Die erfindungsgemäßen Komplexverbindungen enthalten mindestens ein Metallatom oder -ion, insbesondere aus Aluminium, Zink, Magnesium, Titan, Zirkonium, Eisen, Nickel, Cobalt, Chrom, Bor, Kupfer, Platin, Palladium, Osmium, Iridium, Scandium, Cer, Zinn, Mangan, Rhodium oder Ruthenium.

Bevorzugt sind Komplexverbindungen mit weniger als vier Metallzentren, besonders bevorzugt sind solche mit ein oder zwei Metallzentren. Die Metallzentren können dabei mit verschiedenen Metallatomen und/oder -ionen besetzt sein.

Die Herstellung dieser Metall-Ligand-Komplexverbindungen kann in situ durch Reaktion einer Metallverbindung bzw. eins Metallsalzes oder eines entsprechenden Vorkomplexes mit den Liganden der allgemeinen Formeln (I) und (II) erfolgen. Darüber hinaus kann eine Metall-Ligand-Komplexverbindung durch Reaktion einer Metallverbindung bzw. eines Metallsalzes oder eines entsprechenden Vorkomplexes mit den Liganden der allgemeinen Formeln (I) und (II) und anschließende Isolierung gewonnen werden.

Die Komplexverbindungen auf Basis von einem oder mehreren Metallen, bevorzugt aus metallischen Elementen aus der Gruppe Ru, Co, Rh, Ir, Ni, Pd, Pt, Cu, Al, Zn, Mn, Fe, Sc, Ti, Zr, Sn, B, Os, Ce und mindestens einem erfindungsgemäßen Liganden der Formel (I) oder (II) können bereits als Katalysator verwendet werden. Die Komplexverbindungen können aber auch selbst als Edukt zur Herstellung abgewandelter Katalysatoren genutzt werden.

Im folgenden werden einige Ausführungsbeispiele die Erfindung betreffend gegeben:
Allgemeines

Reaktionen luftempfindlicher Verbindungen wurden in einer argongefüllten Glove-Box oder in Standard Schlenkrohren durchgeführt. Lösungsmittel Tetrahydrofuran (THF), Diethylether und Dichlormethan wurden entgast und mittels einer Lösungsmitteltrocknungsanlage (Innovative Technologies) durch Filtration durch eine mit aktiviertem Aluminiumoxid gefüllte Säule absolutiert, Toluol und Pentan wurden zusätzlich durch eine mit einem Kupferkatalysator gefüllte Säule von Sauerstoff befreit.

Die folgenden Beispiele dienen zur Erläuterung der Erfindung. Sie sollen in keiner Weise eine Beschränkung darstellen.

### Beispiel 1: (R)-2-Hydroxy-2'-trifluormethansulfonyl-1,1'-binaphthyl

Unter Argon werden zu einer Lösung aus 3.817 g (13.33 mmol) (R)-2,2'-Dihydroxy-1,1'-binaphthyl in 50 ml absolutem Dichlormethan 1.631 ml (14 mmol) Lutidin und 170.9 mg (1.4 mmol) DMAP gegeben. Es wird auf 0°C gekühlt und mit 5.00 g (14 mmol) N,N-Bis-trifluormethansulfonyl-anilin versetzt. Nach beendeter Zugabe wird langsam auf Raumtemperatur erwärmt und nach 3 Tagen zur Trockene eingeengt. Die Reinigung erfolgt flashsäulenchromatographisch an Kieselgel, Laufmittel: Toluol. Das Rohprodukt wird ohne weitere Charakterisierung weiter umgesetzt.
Ausbeute: 7.12 g (Rohprodukt)

### Beispiel 2: (R)-2-Hydroxy-2'-phenyl-1,1'-binaphthyl

Unter Argon werden zu einer Lösung aus 7.12 g (R)-2-Hydroxy-2'-trifluormethansulfonyl-1,1 '-binaphthyl in 80 ml absolutem Diethylether 138.2 mg (0.26 mmol) Nickel(II)chlorid(dppe) und 17.4 ml (52.12 mmol) Phenylmagnesiumbromid (3molare Lösung in Dichlormethan) gegeben. Es wird 1 h unter Rückfluß und über Nacht bei Raumtemperatur gerührt und durch Zugabe von gesättigter Ammoniumchlorid-Lösung abgebrochen. Es wird mit Diethylether extrahiert, die organischen Phasen mit Natriumhydrogencarbonat-Lösung und mit Wasser gewaschen, über Magnesiumsulfat getrocknet, filtriert und in Vakuum eingeengt. Die Reinigung erfolgt flashsäulenchromatographisch an Kieselgel, Laufmittel: iso-Hexan/Ethylacetat (10:1).
Ausbeute: 4.51 g (braunes Öl)
¹H-NMR (CDCl₃):
δ = 8.00 - 6.90 (m, 17H, Aromat), 5.10 (s, 1H, OH)

### Beispiel 3: (R)-2-Methoxy-2'-phenyl-1,1 '-binaphthyl

Unter Argon werden zu einer Lösung aus 4.51 g (R)-2-Hydroxy-2'-phenyl-1,1'-binaphthyl in 60 ml absolutem Dichlormethan 7.67 ml (43.44 mmol) N,N-Diisopropylethylamin und 3.33 ml (43.44 mmol) Chlormethylmethylether gegeben. Nach 24 h wird die Reaktion durch Zugabe von 10 ml Wasser abgebrochen. Es wird mit Dichlormethan extrahiert, die organischen Phasen mit Wasser gewaschen und über Magnesiumsulfat getrocknet. Es wird filtriert und im Vakuum eingeengt.
Das Produkt wird ohne weitere Reinigung weiter umgesetzt.

### Beispiel 4: (R)-3-Formyl-2-methoxy-2'-phenyl-1,1'-binaphthyl

Unter Argon werden bei -78°C zu einer Lösung aus 13.03 mmol (R)-2-Methoxy-2'-phenyl-1,1 '-binaphthyl in 55 ml absolutem Tetrahydrofuran 17.92 ml (26.06 mmol) n-Buthyllithium (1.6 molare Lösung in Hexan) gegeben. Nach 3 h wird das Reaktionsgemisch mit 5.07 ml (65.15 mmol) Dimethylformamid versetzt und über Nacht auftauend gerührt. Die Reaktion wird durch Zugabe von Ammoniumchlorid-Lösung abgebrochen und mit Dichlormethan extrahiert. Die organischen Phasen werden mit Natriumhydrogencarbonat- Lösung und mit Wasser gewaschen, über Magnesiumsulfat getrocknet, filtriert und im Vakuum eingeengt.
Das Produkt wird ohne weitere Reinigung weiter umgesetzt.

### Beispiel 5: (R)-3-Formyl-2-hydroxy-2'-phenyl-1,1'-binaphthyl

Unter Argon werden zu einer Lösung des Rohproduktes aus Beispiel 4 in 50 ml absolutem Dichlormethan 2.0 g Molekularsieb 4Å und 6.87 ml (4 Äq., 52.82 mmol) Bromtrimethylsilan gegeben. Nach 3 h wird das Reaktionsgemisch mit gesättigter Natriumhydrogencarbonat-Lösung unter Schaumbildung versetzt und mit Dichlormethan extrahiert. Die organischen Phasen werden über Magnesiumsulfat getrocknet, filtriert und im Vakuum eingeengt.
Die Reinigung erfolgt flashsäulenchromatographisch an Kieselgel, Laufmittel: iso-Hexan/Toluol (3:7). Zweite Säule: Kieselgel, Laufmittel iso-Hexan/Ethylacetat (10:1). Beim einengen kristallisiert die Verbindung in Form gelber Kristalle.
Ausbeute: 718 mg gelbe Kristalle (2.07 mmol, 15.9 % über 5 Stufen)
¹H-NMR (CDCl₃):
δ = 10.40 (s, 1H, CHO), 10.10 (s, 1H, OH), 8.20 - 6.90 (m, 16 H, Aromat)

### Beispiel 6: (R)-3-[(2-Hydroxy-phenylimino)-methyl]-2-hydroxy-2'-phenyl-1,1 '-binaphthyl

Unter Argon werden zu 500 mg ausgeheiztem Molekularsieb 187 mg (0.5 mmol) (R)-3-Formyl-2-hydroxy-2'-phenyl-1,1'-binaphthyl in 6 ml absolutem Dichlormethan gegeben und mit 57.3 mg (0.525 mmol) 2-Hydroxyanilin versetzt. Nach 5 Tagen wird über ausgeheizte Cellulose filtriert, mit 2 ml absolutem Dichlormethan eluiert und das Lösungsmittel unter Vakuum entfernt.
Ausbeute: hellbrauner Feststoff (quantitativ)
¹H-NMR (abs. CD₂Cl₂):
δ = 12.26 (s, 1 H, OH), 8.71 (s, 1H, Imin-H), 8.05 - 6.83 (m, 20 H, Aromat)
¹³C-NMR (abs. CD₂Cl₂) 162.16 (Imin-C)

### Katalyseexperimente

### Beispiel 7: Addition von Diethylzink an Benzaldehyd

Unter Argon werden 0.021 mmol (R)-3-[(2-Hydroxy-phenylimino)-methyl]-2-hydroxy-2'-phenyl-1,1'-binaphthyl in 10 ml abs. Toluol gelöst. Zu dieser Lösung werden bei R.T. 115µl Benzaldehyd gegeben und anschließend 20 min gerührt. Die Lösung wird danach auf -30°C abgekühlt und tropfenweise mit 1.5 ml 1M Diethylzink-Lösung versetzt. Die Lösung wird über 60 min auf -5°C erwärmt und weitere 24h bei dieser Temperatur gerührt. Die Reaktionslösung wird mit 10 ml 1M Salzsäure hydrolysiert und die wäßrige Phase dreimal mit je 20 ml Dichlormethan extrahiert. Nach dem Trocknen der organischen Phasen wird das Lösungsmittel entfernt und das Rohprodukt mittels Chromatographie gereinigt. Der Enantiomerenüberschuß wird mit Hilfe chiraler HPLC (Chiralcel OD) bestimmt.

| | |
|---|---|
| Ausbeute: | 85% |
| ee: | 70% |

### Beispiel 8: 1,4-Addition an 2-Cyclohexenon

Unter Argon werden 0.03 mmol (R)-3-[(2-Hydroxy-phenylimino)-methyl]-2-hydroxy-2'-phenyl-1,1 '-binaphthyl und 0.025 mmol Kupfer(II)triflat in 5ml abs. Acetonitril gelöst und 1h bei R.T. gerührt. Anschließend wird die Lösung auf -30°C abgekühlt. Zu dieser Lösung werden 100mg 2-Cyclohexenon und 2.2ml 1M Diethylzink-Lösung gegeben. Die Reaktionslösung wird 24h bei -30°C gerührt, anschließend auf R.T erwärmt, mit 10 ml Diethylether verdünnt und mit 10ml Ammoniumchlorid-Lösung hydrolysiert. Die wäßrige Phase wird dreimal mit 25ml Diethylether extrahiert. Nach dem Trocknen wird das Lösungsmittel entfernt und das Rohprodukt mittels Chromatographie gereinigt. Der Enantiomerenüberschuß wird mit Hilfe chiraler Gaschromatographie (Lipodex A) bestimmt.

| | |
|---|---|
| Ausbeute: | 79% |
| ee: | 57% |

## Patentansprüche

1. Chiraler, unsymmetrischer, mehrzähniger Stickstoff/Sauerstoff-Liganden der Formeln (I) und (II) 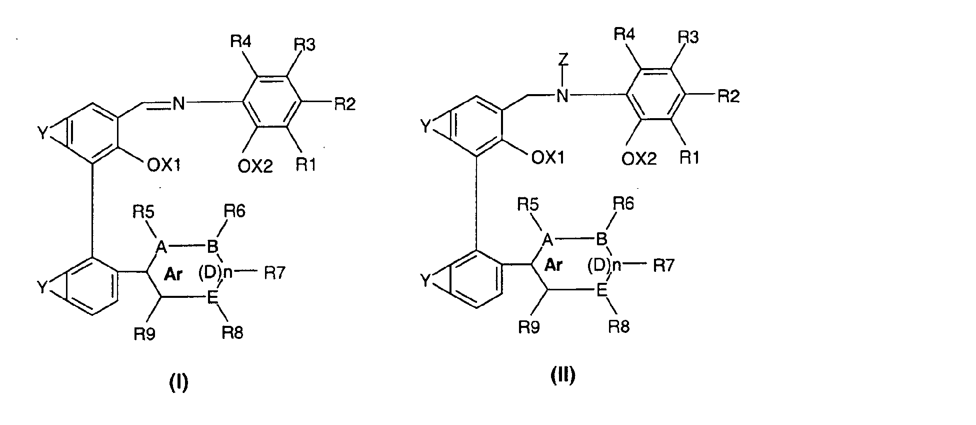 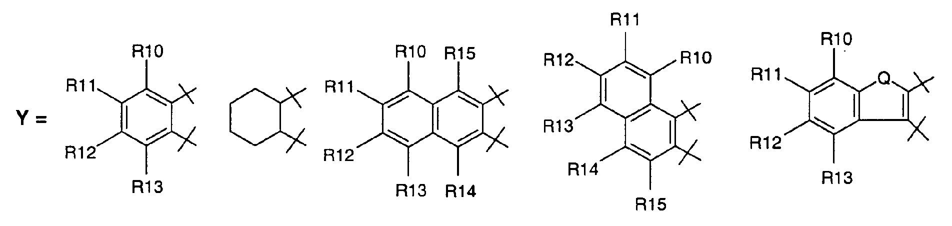 worin n 0 oder 1 sein kann und
Ar Teil eines sechsgliedrigen aromatischen oder 5- bis 6-gliedrigen heteroaromatischen Ringsystems ist, wobei das heteroaromatische Ringsystem entweder 1-3 Stickstoffatome oder 1 Sauerstoffatom oder 1 Schwefelatom in den Positionen A, B, D, E enthalten kann und
R1-R9 unabhängig voneinander ein Wasserstoffatom oder C₁-C₂₀-Alkyl, C₁-C₁₀-Haloalkyl, C₃-C₈-Cycloalkyl, C₅-C₈-Cycloalkenyl, wobei der Cyclus auch 1-2 Heteroatome, ausgewählt aus der Gruppe N, O, S, enthalten kann, C₆-C₁₄-Aryl, Phenyl, Naphthyl, Fluorenyl, C₂-C₁₃-Heteroaryl, wobei die Zahl der Heteroatome, ausgewählt aus der Gruppe N, O, S, 1-4 betragen kann und
wobei die vorgenannten Gruppen wiederum ein- oder mehrfach substituiert sein können, wobei diese Substituenten unabhängig voneinander Wasserstoff, C₁-C₂₀-Alkyl, C₂-C₂₀-Alkenyl, C₁-C₁₀-Haloalkyl, C₃-C₈-Cycloalkyl, C₃-C₈-Cycloalkenyl, C₂-C₉-Heteroalkyl, C₁-C₉-Heteroalkenyl, C₆-C₈-Aryl, Phenyl, Naphthyl, Fluorenyl, C₂-C₆ Heteroaryl, wobei die Zahl der Heteroatome, aus der Gruppe N, O, S, 1-4 betragen kann, C₁-C₁₀-Alkoxy, C₁-C₉-Trihalomethylalkyl, Trifluormethyl, Trichlormethyl, Fluoro, Chloro, Bromo, lodo, Cyano, C₁-C₈-Alkyl oder C₆-Aryl darstellt, oder Tri-(C₁-C₆)-Alkylsilyl sein können, und wobei zwei dieser Substituenten auch verbrückt sein können und worin
Z Wasserstoff, C₁-C₂₄ Alkyl, Benzyl, C₆-C₈Aryl, Phenyl, Naphthyl, Allyl, oder Vinyl sein kann und
X1, X2 unabhängig voneinander Wasserstoff, Methyl, Ethyl, Methoxymethyl, Methoxyethyl sein können und
Q Sauerstoff, Schwefel oder Stickstoff sein kann und worin
R10-R15 unabhängig voneinander ein Wasserstoffatom oder C₁-C₂₀-Alkyl, C₁-C₁₀-Haloalkyl, C₃-C₈-Cycloalkyl, C₅-C₈-Cycloalkenyl, wobei der Cyclus auch 1-2 Heteroatome, ausgewählt aus der Gruppe N, O, S, enthalten kann, C₆-C₁₄-Aryl, Phenyl, Naphthyl, Fluorenyl, C₂-C₁₃-Heteroaryl,
wobei die Zahl der Heteroatome, ausgewählt aus der Gruppe N, O, S, 1-4 betragen kann, sein können,
wobei die vorgenannten Gruppen selbst jeweils ein- oder mehrfach substituiert sein können, wobei diese Substituenten unabhängig voneinander Wasserstoff, C₁-C₂₀-Alkyl, C₂-C₂₀-Alkinyl, C₂-C₂₀-Alkenyl, C₁-C₁₀-Haloalkyl, C₃-C₈-Cycloalkyl, C₃-C₈-Cycloalkenyl, C₂-C₉-Heteroalkyl, C₁-C₉-Heteroalkenyl, C₆-C₈-Aryl, Phenyl, Naphthyl, Fluorenyl, C₂-C₆-Heteroaryl, wobei die Zahl der Heteroatome aus der Gruppe N, O, S 1-4 betragen kann, C₁-C₁₀-Alkoxy, C₁-C₉-Trihalomethylalkyl, Trifluormethyl, Trichlormethyl, Fluoro, Chloro, Bromo, lodo, Cyano, Carboxylato der Formen COOH und COOM' wobei M' entweder ein einwertiges Kation oder C₁-C₄-Alkyl darstellt, C₁-C₆-Acyloxy, Sulfinato, C₁-C₈-Alkyl oder C₆-Aryl darstellt, oder Tri-(C₁-C₆)-Alkylsilyl sein können.

2. Verbindung nach Anspruch 1, **dadurch gekennzeichnet ist, dass** X1 und X2 Wasserstoff sind.

3. Verbindung nach Anspruch 1 oder 2, **dadurch gekennzeichnet, dass** Ar ein Pyridyl-, Furyl-, Thiophenyl- oder Pyrrolyl-Rest ist.

4. Verbindungen nach einem der Ansprüche 1 bis 3, **dadurch gekennzeichnet, dass** die Verbindungen (I) und (II) enantiomerenangereichert sind.

5. Verbindungen nach einem der Ansprüche 1 bis 4, **dadurch gekennzeichnet sind, dass** die Enantiomerenanreicherung 90% übersteigt.

6. Verbindungen nach einem der Ansprüche 1 bis 5, **dadurch gekennzeichnet, dass** die Enantiomerenanreicherung 98% übersteigt.

7. Komplexverbindungen enthaltend mindestens einen Liganden nach Anspruch 1 und mindestens ein Metallatom oder -ion.

8. Komplexverbindung erhältlich durch Umsetzung mindestens eines Metalls, eines Metallsalzes oder eines Metallvorkomplexes mit mindestens einem Liganden gemäß Anspruch 1.

9. Komplexverbindung nach Anspruch 7 oder 8, **dadurch gekennzeichnet, dass** die Verbindungen Aluminium, Zink, Magnesium, Titan, Zirkonium, Eisen, Nickel, Cobalt, Chrom, Bor, Kupfer, Platin, Palladium, Osmium, Iridium, Scandium, Cer, Zinn, Mangan, Rhodium, Ruthenium oder eine Mischung dieser Metalle enthält.

10. Verwendung einer Komplexverbindung nach einem der Ansprüche 7 bis 9 als Katalysator für asymmetrische Reaktionen.

11. Verwendung einer Komplexverbindung nach einem der Ansprüche 7 bis 9 als Katalysator für asymmetrische Transferhydrierungen, Reduktionen mit anorganischen Hydriden, Hydrosilylierungen, Epoxidöffnungen, Epoxidierungen, allylische Oxidationen, Bayer-Villinger Oxidationen, Oxidation von Sulfiden, Cyclopropanierungen, Diels-Alder Reaktionen, Michael-Additionen, Cyanhydrinreaktionen, Strecker und streckerartige Reaktionen, En-Reaktionen, [3+2] Cycloadditionen, Grignard Reaktionen, Addition von Zinkorganylen an Carbonylverbindungen oder Aldol-Reaktionen.

12. Verfahren zur Herstellung von Verbindungen gemäß Anspruch 1 umfassend folgende Verfahrensschritte:
a) Sulfonierung eines 2,2'-Dihydroxy-1,1'-biaromaten der Formel 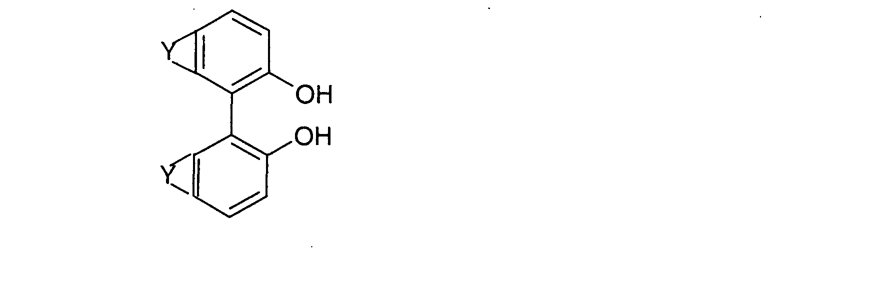 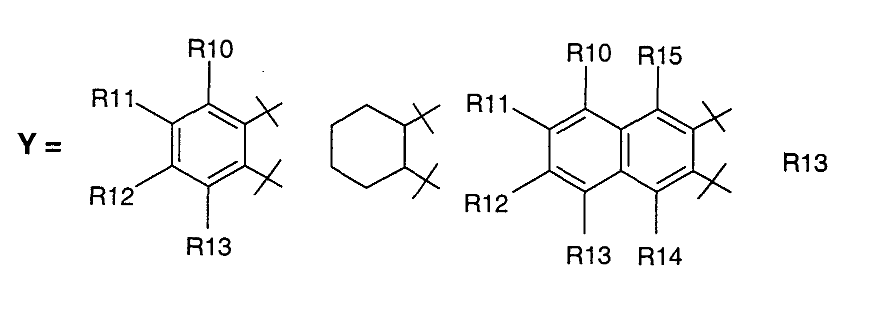 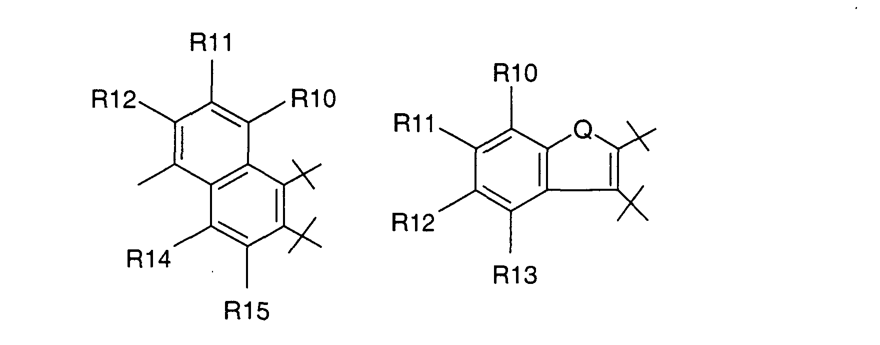 in Anwesenheit einer Base,
b) Kupplung des unter a) erhaltenen sulfonierten Produktes mit einer ArMgBr-Verbindung mit 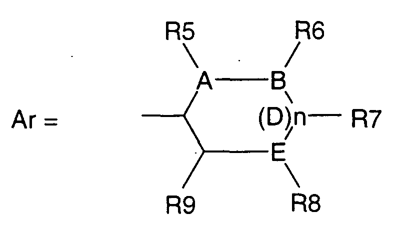 in Gegenwart eines Übergangsmetall-Katalysators,
c) Schützung der freien Hydroxygruppen,
d) Carbonylierung des unter c) erhaltenen Produktes in ortho-Stellung zur geschützen Hydroxygruppe,
e) Entschützung der Hydroxyfunktion und Umsetzung des carbonylierten Produktes aus d) mit einem aromatischen Amin der Formel

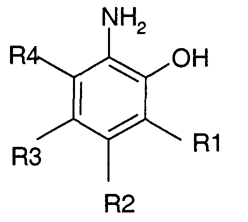

13. Verfahren nach Anspruch 12, **dadurch gekennzeichnet, dass** die Umsetzung in Schritt e) eine reduktive Aminierung ist.

14. Verfahren nach Anspruch 12, **dadurch gekennzeichnet, dass** die Umsetzung in Schritt e) eine Aminierung mit anschließender Reduktion ist.
